# EUROPEAN PATENT SPECIFICATION

(11) **EP 0 807 124 B1**
(45) Date of publication and mention of the grant of the patent: **17.05.2006**
(21) Application number: 95922533.5
(22) Date of filing: 09.06.1995
(51) Int. Cl.: C07K 16/28, C12N 5/20, C12Q 1/68, G01N 33/577, A61K 39/395, A61K 49/00, C07K 14/71

(54) **MONOCLONAL ANTIBODY AGAINST FLT4 RECEPTOR TYROSINE KINASE AND ITS USE IN DIAGNOSIS AND THERAPY**
MONOKLONALER ANTIKÖRPER GEGEN FLT4-REZEPTOR-TYROSINKINASE UND DESSEN VERWENDUNG ZUR DIAGNOSE UND THERAPIE
ANTICORPS MONOCLONAL CONTRE LE RECEPTEUR DE LA TYROSINE KINASE FLT4 ET SON UTILISATION COMME AGENT DE DIAGNOSTIC ET COMME AGENT THERAPEUTIQUE

(30) Priority: 09.06.1994 US 257754
(43) Date of publication of application: 19.11.1997
(73) Proprietor: Licensing Oy, 00130 Helsinki (FI); Orion Corporation, 02101 Espoo (FI)
(72) Inventor: Alitalo, Kari, c/o Molecular/Cancer Biology Lab., 00014 Helsinki (FI); Kaipainen, Arja, 00250 Helsinki (FI); Korhonen, Jaana, 00530 Helsinki (FI); Mustonen, Tuija, 00270 Helsinki (FI); Matikainen, Marja-Terttu, 23100 Mynämäki (FI); Pajusola, Katri, 00900 Helsinki (FI); Karnani, Päivi, c/o Molecular/Cancer Biology Lab., 00014 Helsinki (FI)
(74) Representative: Vossius & Partner
(86) International application number: PCT/FI1995/000337
(87) International publication number: WO 1995/033772

(56) References cited:
- WO-A-93/14124
- WO-A-94/10202
- ONCOGENE, vol. 8, no. 11, November 1993 BASINGSTOKE, GB, pages 2931-2937, K. PAJUSOLA ET AL. 'Two human FLT4 receptor tyrosine kinase isoforms with distinct carboxy terminal tails are produced by alternative processing of primary transcripts.'
- ONCOGENE, vol. 8, no. 5, May 1993 BASINGSTOKE, GB, pages 1233-1240, F. GALLAND ET AL. 'The FLT4 gene encodes a transmembrane tyrosine kinase related to the vascular endothelial growth factor receptor.' cited in the application
- CANCER RESEARCH, vol. 52, no. 20, 15 October 1992 BALTIMORE, MD, USA, pages 5738-5743, K. PAJUSOLA ET AL. 'FLT4 receptor tyrosine kinase contains seven immunoglobulin-like loops and is expressed in multiple human tissues and cell lines.' cited in the application
- THE JOURNAL OF EXPERIMENTAL MEDICINE, vol. 178, no. 6, 1 December 1993 NEW YORK, NY, USA, pages 2077-2088, A. KAIPAINEN ET AL. 'The related FLT4, FLT1, and KDR receptor tyrosine kinases show distinct expression patterns in human fetal endothelial cells.' cited in the application
- PROCEEDINGS OF THE NATIONAL ACADEMY OF SCIENCES OF THE USA, vol. 92, no. 8, 11 April 1995 WASHINGTON, DC, USA, pages 3566-3570, A. KAIPAINEN ET AL. 'Expression of the fms-like tyrosine kinase 4 gene becomes restricted to lymphatic endothelium during development.'
- FERRERA N. ET AL: "The biology of VEGF" ENDOCRINE REVIEWS, vol. 18, no. 1, 1997, pages 4-25,
- B. PYTOWSKI ET AL.: "Complete and specific inhibition of adult lymphatic regeneration by a novel VEGFR-3 Neutralizing Antibody" JOURNAL OF THE NATIONAL CANCER INSTITUTE, vol. 97, no. 1, 5 January 2005 (2005-01-05), pages 14-21,
- L.CHEN ET AL.: "Vascular endothelial growth factor receptor-3 mediates induction of corneal alloimmunty" NATURE MEDICINE, vol. 10, no. 8, August 2004 (2004-08), pages 813-815,
- K. SHIMIZU ET AL.: "Suppression of VEGFR-3 signaling inhibits lymph node metastasis in gastric cancer" CANCER SCIENCE, vol. 95, no. 4, April 2004 (2004-04), pages 328-333,
- H. KUBO ET AL.: "Blockade of vascular endothelial growth factor receptor-3 signaling inhibits fibroblast growth factor-2-induced lymphangiogenesis in mouse cornea" PNAS, vol. 99, no. 13, 25 June 2002 (2002-06-25), pages 8868-8873,
- M. CHOY ET AL.: "VEGF-C signaling through Flt-4 (VEGFR3) medaites leukemic cell proliferation and survival" BLOOD, vol. 96, no. 11, 16 November 2000 (2000-11-16), pages 502A-503A,
- X. ZHANG ET AL.: "The Vegfr-3 receptor participates in Kaposi's sarcoma associated virus (KSHV)/human herpes virus 8 (HHV-8) infection of endothelial cells" BLOOD, vol. 102, no. 11, 16 November 2003 (2003-11-16), page 279A,
- P. SAHARINEN ET AL.: "Lymphatic vasculature: development, molecular regulation and role in tumor metastasis and inflammation" TRENDS IN IMMUNOLOGY, vol. 25, no. 7, July 2004 (2004-07), pages 387-395,
- C. CURSIEFEN ET AL.: "Spontaneous corneal hem- and lymphangiogenesis in mice with destrin-mutation depend on VEGFR3 signaling" AMERICAN JOURNAL OF PATHOLOGY, vol. 166, no. 5, May 2005 (2005-05), pages 1367-1377,

## Description

### FIELD OF THE INVENTION

The present invention relates generally to receptor tyrosine kinases, nucleic acid probes and antibodies specifically recognizing such receptors, and the use of such probes and antibodies for identifying lymphatic vessels and high endothelial venules (HEV) in animal and human tissues and lymphatic endothelial cells in culture. More specifically the present invention is directed to antibodies specific to FLT4, a receptor tyrosine kinase, and to methods for identifying FLT4 expression in lymphatic vessels and ultimately diagnosing and treating disease states in animals and humans involving changes in lymphatic tissue, such as inflammatory, infectious and immunological diseases, metastatic lymph nodes and lymphangiomas.

### BACKGROUND OF THE INVENTION

The physiology of the vascular system, embryonic vasculogenesis and angiogenesis, blood clotting, wound healing and reproduction, as well as several diseases, involve the vascular endothelium lining the blood vessels. The development of the vascular tree occurs through angiogenesis and, according to some theories, the formation of the lymphatic system starts shortly after arterial and venous development by sprouting from veins ^{(1, 2)}.

After the fetal period endothelial cells proliferate very slowly, except during angiogenesis associated with neovascularization. Growth factors stimulating angiogenesis excert their effects via specific endothelial cell surface receptor tyrosine kinases.

The protein product of the FLT4 receptor tyrosine kinase cDNA, cloned from a human erythroleukemia cell line is N-glycosylated and contains seven immunoglobulin-like loops in its extracellular domain. The cytoplasmic tyrosine kinase domain of FLT4 is about 80 % identical at the amino acid level with the corresponding domains of FLT1 and KDR and about 60 % identical with the receptors for platelet-derived growth factor, colony stimulating factor-1, stem cell factor and the FLT3 receptor ⁽³⁾.

Although the biological function of FLT4 are as yet unknown, its restricted expression pattern indicated that its functions may involve the vascular endothelium. Our previous results revealed FLT4 mRNA expression in endothelial cells in developing vessels of several fetal organs as disclosed by Kaipainen et al., in *J. Exp. Med.* 178: 2077-2088, 1993. A comparison of FLT4, FLT1, and KDR/FLK-1 receptor mRNA signals showed overlapping, but distinct expression patterns in the tissues studied ⁽⁴⁾. These data suggested that the receptor tyrosine kinases encoded by this gene family may have distinct functions in the regulation of the growth and/or differentiation of blood vessels.

A major function of the lymphatic system is to provide fluid return from tissues and transport many extravascular substances back to the blood. In addition, during the process of maturation, lymphocytes leave the blood, migrate through lymphoid organs and other tissues, and enter the lymphatic vessels, and return to the blood through the thoracic duct. Specialized venules, high endothelial venules, (HEVs) bind lymphocytes again and cause their extravasation into tissues. The lymphatic vessels and especially the lymph nodes thus play an important role in immunology and they are also sites of development of metastasis of different tumors.

Since the turn of the 20th century, three different theories concerning the embryonic origin of the lymphatic system have been presented. However, prior to the present invention, lymphatic vessels have been difficult to identify, because there are no specific markers available for them.

Lymphatic vessels are most commonly studied with the aid of lymphography. In lymphography, X-ray contrast medium is injected directly into a lymphatic vessel. That contrast medium is distributed along the efferent drainage vessels of the lymphatic system. The contrast medium is collected in lymph nodes, where it stays for up to half a year, during which time X-ray analyses allow the follow-up of lymph node size and architecture. This diagnostics is especially important in cancer patients with metastases in the lymph nodes and in lymphatic malignancies, such as lymphoma

### SUMMARY OF THE INVENTION

The present application is directed to FLT4 peptides and other constructs and to the use of FLT4 as a specific marker for lymphatic endothelial cells.

The invention is directed to antibodies specifically recognizing FLT4, especially to monoclonal antibodies, and compostions containing such antibodies. Further disclosed in the present application is the use of such monoclonal antibodies for diagnostic purposes for detecting and measuring the amount of FLT4 receptors in tissues, especially in lymphatic tissues and in lymphatic endothelial cells.

In a preferred embodiment, the invention provides monoclonal antibodies specifically recognizing the FLT4 receptor. More specifically this invention provides a monoclonal antibody designated 9D9F9. The hybridoma cell line which produces monoclonal antibody 9D9F9 is deposited with the Deutsche Sammlung von Mikroorganismen und Zellkulturen GmbH (DSM) under the provisions of the Budapest Treaty (DSM accession number ACC2210).

Monoclonal antibodies labelled with a detectable marker are also provided. As used herein, the term detectable marker encompasses any detectable marker known to those skilled in the art. However, in a preferred embodiment of this invention, the detectable marker is selected from the group consisting of radioisotopes, florochromes, dyes, enzymes and biotin. For the purpose of this invention suitable radioisotopes include, but are not limited to ¹²⁵I and ¹³¹I.

Monoclonal antibodies of the present invention may also be used in a method for detecting the presence of FLT4-receptors in a cell sample, comprising the steps of exposing a cell sample to a monoclonal antibody of the present invention and detecting the binding of said monoclonal antibody to FLT4-receptors.

Another aspect of the present invention thus relates to a method of determining the presence of FLT4-receptors in a cell sample, comprising the steps of:
(a) exposing a cell sample to a monoclonal antibody of the present invention;
(b) detecting the binding of said monoclonal antibody to FLT4-receptors.

The exposure of a cell mixture to monoclonal antibodies of the invention can be in solution, as is the case for fluorescence-activated cell sorting, or it can be on solid tissue specimens, such as biopsy material, or it can be with the monoclonal antibody immobilized on a solid support, as is the case with column chromatography or direct immune adherence. The mixture of cells that is to be exposed to the monoclonal antibody can be any solution of blood cells or tissue cells. Prefereably, the cell mixture is from normal or pathological tissue containing or suspected to contain lymphatic endothelial cells. After exposure of the cell mixture to the monoclonal antibody, those cells with FLT4 -receptors will bind to the monoclonal antibody to form an antibody-FLT4 -receptor complex. The presence of the antibody-FLT4 -receptor complex, and therefore FLT4 receptors, can be detected by methods known in the art. These methods include immunohistochemical methods standard in the art, such as immunofluorescence, FACS analysis, ELISA, IRMA (a sandwich type of immunochemistry assay), immunohisto-chemistry, RIA using ¹²⁵I-label and autoradiography.

The present invention also provides monoclonal antibodies conjugated to an imageable agent. As used herein, the term imageable agent includes, but is not limited to, radioisotopes. A preferred radioisotope is 99m-technetium.

In a specific embodiment, the invention is directed to a method for monitoring lymphatic vessels and their endothelial cells in tissue samples and in organisms. The present invention further provides clinical detection methods describing the state of lymphatic tissue, ans especially lymphatic vessels (inflammation, infection, traumas, growth, neoplasia etc.) and methods for detecting lymphatic vessels and thus lymphatic vascularization in an organism.

More specifically the present invention provides a method for detecting and identifying lymphatic changes charactherized by FLT4 expression in connection to metastatic cancers, inflammatory, infectious and immunological conditions, which method comprises the steps of
(a) obtaining a tissue and/or body fluid sample suspected of lymphatic changes, and
(b) contacting said sample with a FLT4-specific monoclonal antibody under conditions suitable for forming a complex between the monoclonal antibody and the antigen, and
(c) detecting the prescence of any complex formed.

A tissue which may be detected by this method is any normal, precancerous or cancerous solid tumor tissue with FLT4-containing lymphatic cells or cells which express the FLT4-receptor. In one embodiment of the present invention, the monoclonal antibody is labelled with a detectable marker as described herein. Methods of the invention are useful for detecting and differentiating various forms of cancer, especially metastases in the lymph nodes and other lymphatic malignancies, such as lymphomas, as well as lymphangiomas.

A method of imaging the presence of lymphatic vessels, high endothelial venules or lymph nodes in human patients, is also provided by this invention. This method comprises administration of labelled antibodies and detection by imaging at sites where FLT4 expressing cells are present, in lymphatic vessels or lymph nodes.

The invention is further directed to a method of stimulating or antagonizing the function of FTL4 in lymphatic vascularization and in inflammatory, infectious and immunological conditions, said method comprising inhibiting the FLT4-mediated lymphatic vascularization by providing amounts of a FLT4-binding compound sufficient to block the FLT4 endothelial cell sites participating is such reaction, especially where FLT4 function is associated with a disease such as metastatic cancers, lymphomas, inflammation (chronic or acute), infections and immunological diseases.

### BRIEF DESCRIPTION OF THE DRAWINGS

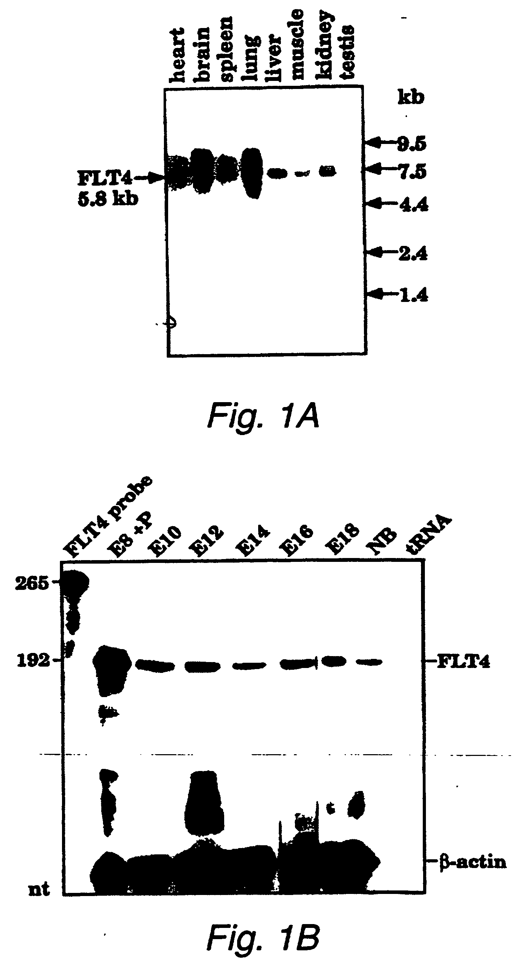

Figure 1. Expression of FLT4 mRNA in mouse tissues A. Hybridization of polyadenylated RNA isolated from the indicated tissues of adult mice. The size of the FLT4 mRNA band is given in kilobases. B. RNAse protection analysis of RNA isolated from mouse embryos of various gestational ages (E8-E18) and from a newborn mouse (1 day). Sample E8+P contains also the placenta. The size of the probe and the protected FLT4 fragment are given in nt; β-actin was used as a control.

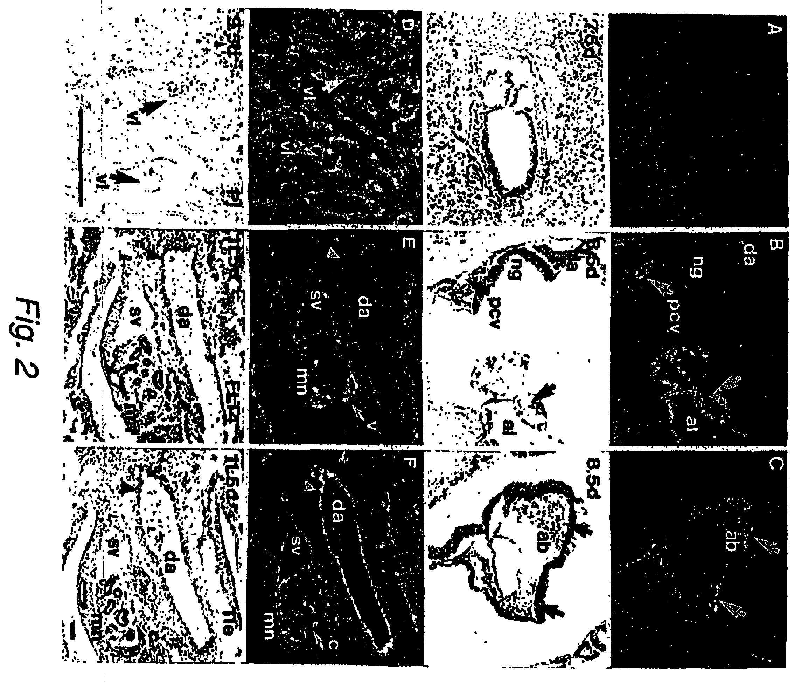

Figure 2. Expression of FLT4 mRNA in a 7.5-, 8.5- and 11.5-day p.c. embryos. Darkfield and brightfield photomicrographs of *in situ* autoradiograms are shown. No expression of FLT4 mRNA could be detected in a 7.5-day embryo (A). The FLT4 expression of an 8.5-day p.c. mouse embryo is shown in (B) and (C). Arrows point to FLT4 positive cells in the endothelium of posterior cardinal vein (cv), in the allantois (al) in (B) and in angioblasts (ab) of the head mesenchyme in (C). In a 8,5-day p.c. placenta FLT4 transcripts can be seen in endothelial cells of venous lacunae (vl) (D). Panels E and F show a comparison of FLT4 and Tie hybridization signals in 11.5-day p.c. embryos. The region of the developing dorsal aorta and metanephros (mn) is shown (20x). Note that the dorsal aorta is negative for FLT4, but positive for Tie mRNA, whereas both probes hybridize with the endothelium of the subendocardial vein (sv). Also, the FLT4 probe gives a signal from the metanephric vein (v), whereas Tie mostly hybridizes with the metanephric capillaries (c, arrows). da: dorsal aorta, ng: neural groove. Scale bar: 30 µm.

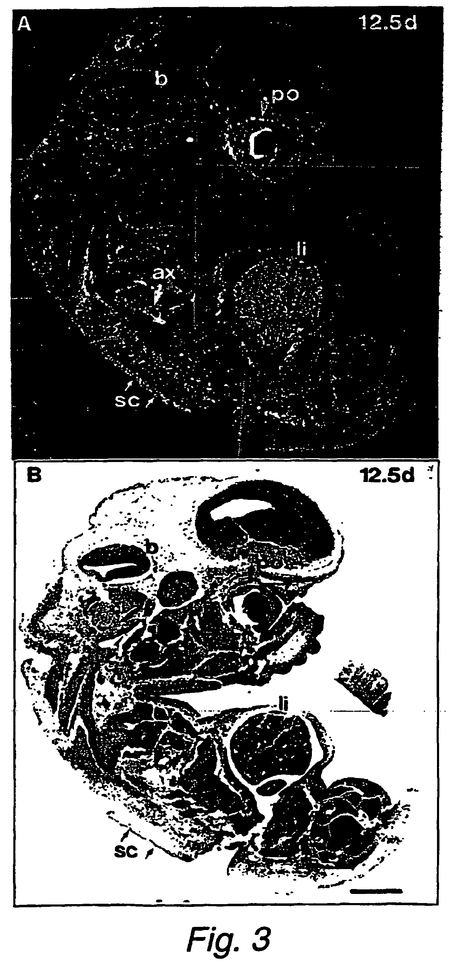

Figure 3. FLT4 mRNA expression in a 12.5-day p.c. embryo. A sagittal section through the axillar plane is shown. Note that FLT4 mRNA is prominent in dilated vessels of the axilla (ax), in a plexus-like pattern in the periorbital (po) region, in the paravertebral tissue (arrowheads) and in the subcutaneous (sc) tissue. b: brain, li: liver. Scale bar: 5 µm.

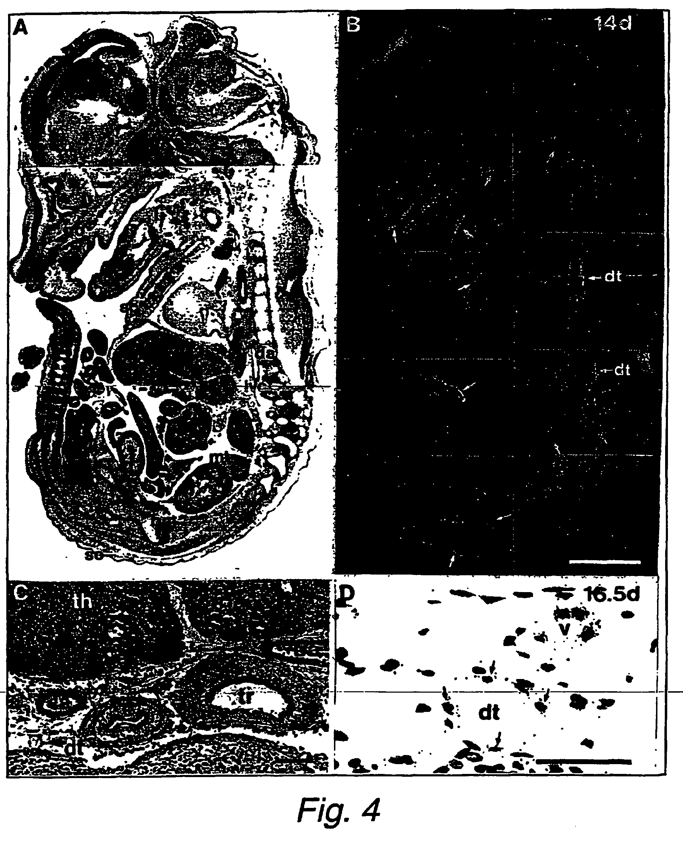

Figure 4. FLT4 in 14 and 16.5 day p.c. embryos. Panels A and B show bright and darkfield images of a midsagittal section. po: periorbital region, lj: lower jaw, ne: neck region, sc: subcutis, mt, mesenterium, ao, aorta. dt: thoracic duct. (C) shows a transverse section of a 16.5 day embryo in hematoxylin-eosin staining. th: thymus, tr: trachea, e: esophagus, ca: carotid artery, ba: brachiocephalic artery, dt: thoracic duct. (D) shows a higher magnification (40x) of the region of ductus thoracicus; the autoradiographic grains can be seen over the endothelial cells. Also, the small vessel (v) in the upper part of the photograph is positive. Scale bar: 10 µm (A-C), 1µm (D).

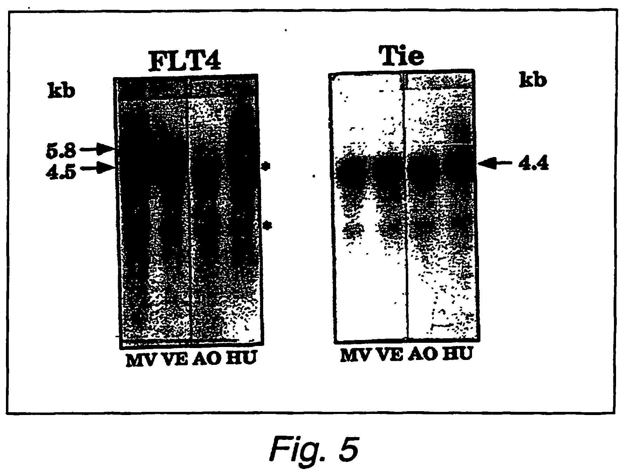

Figure 5. Comparison of FLT4 and Tie mRNA expression in cultured endothelial cells. Northern blot analysis of polyadenylated RNA from human foreskin microvascular (MV), femoral vein (VE), aortic (AO) and umbilical vein (HU) endothelial cells. For a comparison, the hybridization signal of the Tie receptor tyrosine kinase mRNA is shown. The bands resulting from the unspecific binding of the probe to the ribosomal RNA are marked with asterixes.

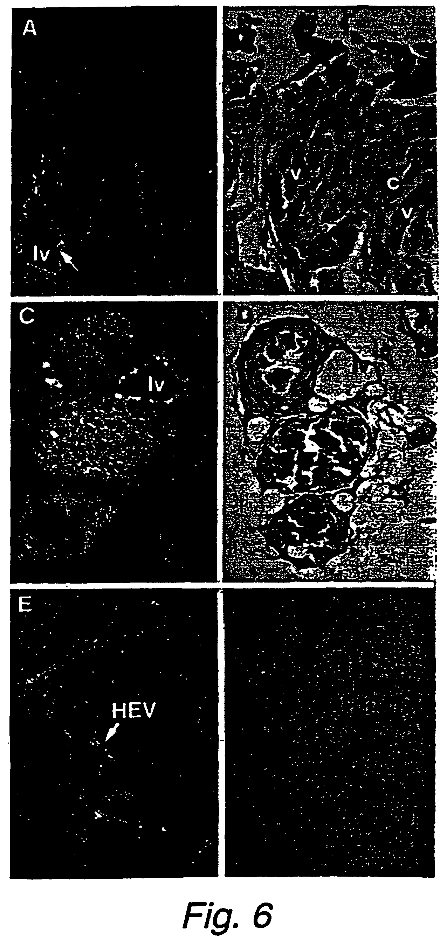

Figure 6. FLT4 in adult human lymphatic vessels of the mesenterium (A,B), lung (C,D), and tonsil (E,F). Note that only the lymphatic vessels in A and C give a FLT4 signal, whereas the veins, capillaries and the arteries are negative for FLT4 mRNA. In the tonsil, the signal is found in the endothelia of some HEVs. Scale bar: 200 µm.

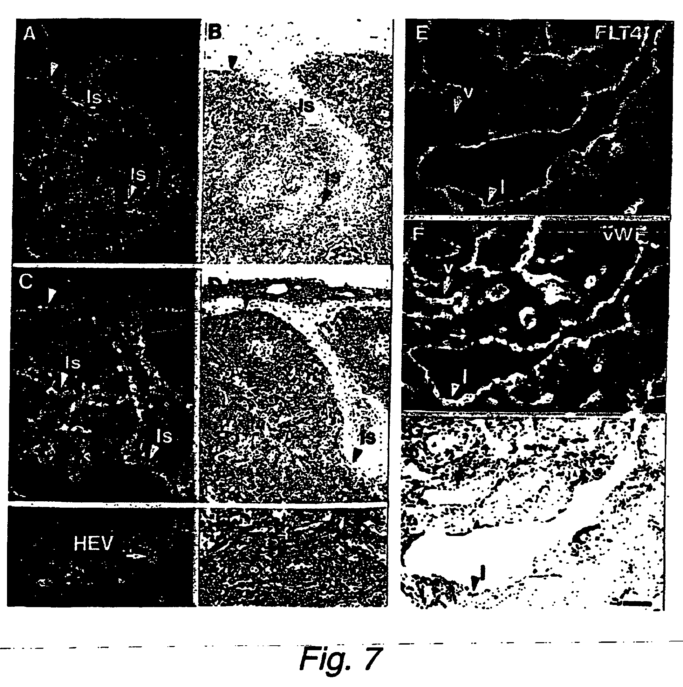

Figure 7. FLT4 mRNA in normal (A, B) and metastatic (C, D) lymph node and in lymphangioma (E-G). Arrowheads mark the lymphatic sinuses and HEVs, which are FLT4 positive. A comparison of FLT4 and von Willebrand factor signals shows both in the lymphatic endothelium, but only von Willebrand factor signal in the capillary (c) and venous (v) endothelia. Scale bars: 10 µm (A-D) and 100 µm (E-G).

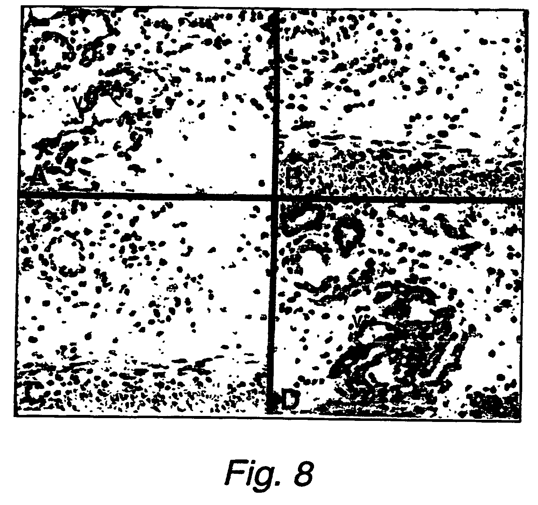

Figure 8. FLT4 expression in fetal mesenterial vessels detected by immunoperoxidase staining. Sections were stained with affinity-purified anti-FLT4 antibodies (A), with antigen-blocked antiserum (B) and with preimmune serum (C) and with antiserum specific against the factor VIII-related antigen (D). Note that staining is confined to some, but not all vessels (v). Scale bar, 0.05 mm.

### DETAILED DESCRIPTION OF THE INVENTION

Recognizing the importance of identifying changes in lymphatic tissues, especially in lymph nodes in connection to metastatic cancers and immunological disease states, the present inventors have shown that FLT4 is a specific marker that detects lymphatic vessel endothelium and therefore useful as a marker for lymphatic changes in pathological states in man.

The present inventors have earlier shown that the expression pattern of FLT4 in comparison to FLT1 and KDR differs greatly in tissues of 18-week-old human fetuses ⁽⁴⁾. In order to understand the role of FLT4 during development, the inventors cloned partial cDNAs for mouse FLT4. Using these probes in *in situ* hybridization, FLT4 mRNA expression during mouse development was analysed and it was found that FLT4 is expressed during vasculogenesis and angiogenesis of the lymphatic system. The relevance of these fingings was also confirmed in normal and pathological human adult tissues, as FLT4 was found in lymphatic endothelial cells of human adult tissues both in normal and pathological conditions, as well as in some high endothelial venules (HEVs).

The cloning of mouse FLT4 cDNA fragments showed that their deduced amino acid sequence is almost identical with the corresponding human sequence (amino acid identity about 96 % in both segments studied). Further evidence for the identity of the mouse FLT4 cDNA was obtained from Northern hybridization where probes from both species yielded the typical 5.8 kb mRNA signal from mouse tissues. Analysis of RNA isolated from various tissues of adult mice showed FLT4 expression in the liver, lung, heart, spleen and kidney, with no or very little hybridization in the brain and testes. This pattern is similar to the pattern reported earlier by Galland et al. ⁽⁵⁾. The results of RNase protection suggested that the FLT4 gene is needed during mouse development, starting from 8.5 day p.c. embryos, and the relative expression levels appeared quite stable.

For the *in situ* hybridization two fragments of mouse FLT4 cDNA were selected, which encode sequences of the extracellular domain. This allowed a clear distinction of the hybridization pattern from the related FLK-1 and FLT-1 receptor patterns, which show only a very low degree of sequence identity with FLT4 in the extracellular region ^{(6, 7, 8, 9)}. FLT4, similarly to the FLK-1, FLT-1, Tie and Tek endothelial receptor tyrosine kinase genes was not expressed in 7.5 day p.c. embryos. In a 8.5-day p.c. embryo the strongest FLT4 signals were localised in the allantois, the angioblasts of head mesenchyme and the cardinal vein. In contrast, the the dorsal aorta, endocardium of the heart and angioblasts of the yolk sac were negative, unlike for Tie, Tek, FLK-1 and FLT-1, Tie and Tek ^{(10, 8)}. The restriction of FLT4 expression to the venous system was even more clear in samples from 11.5 day mouse embryos; where the Tie mRNA was expressed also in arteries. In 12.5-day p.c. embryos the FLT4 signal decorated developing venous and presumptive lymphatic endothelia, but unlike for the endothelial Tie receptor tyrosine kinase, arterial endothelia were negative. During later stages of development FLT4 mRNA became restricted to vascular plexuses devoid of blood cells, representing developing lymphatic vessels. Only the lymphatic endothelium and some high endothelial venules expressed FLT4 mRNA in adult human tissues. Increased expression occurred in lymphatic sinuses and high endothelial venules in metastatic lymph nodes and in lymphangioma.

Due to difficulties in the interpretation of data from mouse embryos, human endothelia were studied, because the lymphatic system is much better defined in humans. Also, cells established from various endothelia could be studied in cell culture to see if the specificity of FLT4 expression persists in *in vitro* conditions. Endothelial cells lines are known to lose differentiated features upon in vitro culture. Therefore, it was not unexpected that they were negative for FLT4. Cultured aortic endothelial cells were also devoid of FLT4 mRNA. However, signals were obtained from human endothelial cells grown from the microvasculature and from femoral and umbilical veins. Thus, at least some of the specificity of FLT4 expression was retained in cell culture.

*In situ* hybridization analysis of adult human tissues confirmed the restriction of FLT4 to the lymphatic system seen in the developing mouse embryos. FLT4 expression was seen in the lymphatic endothelia and in the sinuses of human lymph nodes. Interestingly, also some of the HEVs, which have a cuboidal endothelium, shown to function in the trafficking of leukocytes to the lymph nodes, were FLT4 positive. Furthermore, a parallel hybridization analysis showed that FLT4 mRNA levels were enhanced in these structures in metastatic as compared to normal lymph nodes. FLT4 was also very prominent in lymphangiomas, which are benign tumours composed of connective tissue stroma and growing, endothelial-lined lymphatic channels. FLT4 mRNA was restricted to the lymphatic endothelium of these tumors and absent from their arteries, veins and capillaries. In the human lung we were able to identify lymphatic structures, which were the only FLT4 positive vessels in this tissue.

The foregoing results suggest that FLT4 is a novel marker for lymphatic vessels and some high endothelial venules in human adult tissues. They also support the theory on the venous origin of lymphatic vessels. FLT4, as a growth factor receptor, may be involved in the differentiation and functions of these vessels.

These results, combined with the FLT4-binding compounds according to the present invention, allows a selective labelling of lymphatic endothelium, especially by using antibodies of the present invention coupled to radioactive, electron-dense or other reporter substances, which can be visualized. It may be possible to inject into the lymphatic system substances, containing FLT4 receptor internalization-inducing monoclonal antibodies, and thereby transport predefined molecules into the lymphatic endothelium. Also, it may be possible to use such the FLT4-binding compounds according to the invention for the detection of high endothelial venules, especially activated HEVs, which express enhanced levels of the FLT4 receptor. To our knowledge, no such specific markers are currently available for lymphatic endothelium.

The following examples are given merely to illustrate the present invention and not in any way to limit its scope.

### EXAMPLES

### EXAMPLE 1

### Cloning of mouse FLT4 cDNA probes

Approximately 10⁶ plaques from a IFIX® II genomic library from 129SV mice (Stratagene) was screened with the S2.5 human FLT4 receptor cDNA fragment covering the extracellular domain ⁽³⁾. A 2.5 kb Bam HI fragment was subcloned from a positive plaque and sequenced from both ends. From this subclone, polymerase chain reaction was used to amplify and clone into the pBluescript KSII+/- vector (Stratagene) an exon fragment covering nucleotides 1745-2049 of the mouse FLT4 cDNA sequence ⁽⁹⁾.

A second fragment covering nucleotides 1-192 was similarly cloned.

### EXAMPLE 2

### Analysis of FLT4 mRNA in mouse tissues

Total RNA was isolated from developing embryos (8-18 days p.c. and one day old mice) according to Chomczynski et al. ⁽¹¹⁾. The sample from 8 day p.c. embryos included also the placenta.

For RNase protection analysis, RNA probe was generated from the linearized FLT4 plasmid obtained according to Example 1 using [³²P]-UTP and T7 polymerase for the antisense orientation. The β-actin probe used corresponds to nucleotides 1188-1279 of the published mouse β-actin sequence ⁽¹²⁾. After purification in a 6% polyacrylamide/7M urea gel, the labelled transcripts were hybridzed to 30 µg of total RNA overnight at 52 °C. Unhybridized RNA was digested with RNase A (10 U/ml) and T1 (1 µg/ml) at 37 °C, pH 7.5 for 1 h. The RNases were inactivated by proteinase K digestion at 37 °C for 15 min and the samples were analysed in a 6% polyacrylamide/7M urea gel.

The pattern of expression of FLT4 analysed in this experiment showed that very weak mRNA signals were obtained from lung, liver, heart, kidney, skeletal muscle and spleen, whereas testis and brain were apparently without specific signal (Fig. 1A). Analysis of a series of RNAs collected during different phases of mouse development by RNase protection assay showed that the FLT4 mRNA was expressed throughout embryogenesis from day 8 p.c. to newborn mice without great variations in signal intensity (Fig. 1B).

### EXAMPLE 3

### In situ hybridization for FLT4 in mouse embryos

To better assign FLT4 transcripts to cells and tissues, sections of 7.5 and 8.5 day p.c. mouse embryos were hybridized with labelled FLT4 RNAs. Mouse embryos were derived from matings of CBA and NMRI mice. Pregnant mice were killed by cervical dislocation and the embryos were either immediately frozen or transferred via phosphate buffered saline into 4% paraformaldehyde. The embryos and isolated mouse organs were fixed for 18 h at 4°C, dehydrated, embedded in paraffin and cut into 6 µm sections.

RNA probes (antisense and sense) of 192 and 349 nucleotides (see Example 1) were generated from linearized plasmids using [³⁵S]-UTP. *In situ* hybridization of sections was performed according to Wilkinson et al. ^{(13, 14)}, with the following modifications: 1) instead of toluene, xylene was used before embedding in paraffin wax, 2) 6 µm sections were cut, placed on a layer of diethyl pyrocarbonate-treated water on the surface of glass slides pretreated with 2% 3-triethoxysilyipropylamine, 3) alkaline hydrolysis of the probes was omitted, and 4) the high stringency wash was for 80 min at 65°C in a solution containing 30 mM DTT and 1 x SSC. The sections were covered with NTB-2 emulsion (Kodak) and stored at 4°C. The slides were exposed for 14 days, developed and stained with hematoxylin. Control hybridizations with sense strand and RNase A-treated sections did not give a specific signal above background.

As shown in Figures 2A and B, FLT4 mRNA was not expressed in 7.5 day p.c. mouse embryos, but bright signals were detected in the posterior cardinal vein (cv) on day 8.5 of development. In contrast, the developing heart (data not shown) and dorsal aorta (da) were FLT4-negative. In the extraembryonic tissues FLT4 was prominently expressed in the allantois (al in panel B), whereas developing blood islands of the yolk sac were negative (data not shown). On the other hand, angioblasts (ab) of the head mesenchyme were strongly FLT4 positive (C). In the developing placenta FLT4 signal was first seen in peripheral sinusoidal veins (data not shown). In 9.5 day p.c. placenta the endothelium of venous lacunae (vl in D) and the giant cells partially fused to the Reichert's membrane (data not shown) expressed FLT4 mRNA.

Thus, although FLT4 expression was very prominent in the earliest endothelial cell precursors, the angioblasts, it appeared to be restricted only to certain vessels of 8.5 day p.c. embryos. The Tie receptor is known to be expressed in all endothelial cells of developing mouse embryos and thus provides a marker for these cells. Notably, in contrast to the Tie probe, the FLT4 probe hybridized very weakly if at all with arterial endothelia of 11.5 day p.c. embryos, e.g. with the endothelium of the developing dorsal aorta (da in Fig. 2 E,F) or the carotic arteries (data not shown). Instead, FLT4 signal was much more prominent in the developing veins. For example, FLT4 signal was detected veins surrounding the developing metanephros (v, sv in E), while the Tie probe predominantly recognized capillaries (c) within the metanephros (F).

As can be seen from Figure 3, FLT4 mRNA is distributed in several regions of a 12.5 day p.c. mouse embryo, being particularly prominent in the dilated vessel of the axillar region (ax). A similar FLT4 positive vessel structure was seen in the mid-sagittal section in the jugular area (data not shown). A plexus-like pattern of FLT4 expressing vessels appeared in the periorbital region (po) and surrounding the developing vertebrae (vb). Also, just beneath the developing skin, a FLT4-positive vascular network was evident (sc). Weaker capillary signals were obtained from several regions, including the developing brain (b). FLT4 mRNA could also be detected in small vessels of the neck region, of the developing snout and at the base of the developing tongue as well as in the tail region (data not shown). Besides, the liver (li) was strongly positive for FLT4 mRNA in a spotlike pattern.

During further development, FLT4 RNA appeared to become more restricted to certain vessels of the embryo. A 14.5 day p.c. embryo shows nicely this restricted pattern of expression (Fig. 4 A,B). In the midsagittal section of Figure 4 the most prominent FLT4 signal is seen along the developing vertebral column in its anterior part. This signal was considered to originate from endothelial cells of the thoracic duct (dt), which is the largest lymphatic vessel formed at this time of development. In contrast, the dorsal aorta (da) and inferior vena cava (vc) were negative. Dilated vessels in the mesenteric region were also strongly positive for FLT4. Furthermore, as in the 12.5 day p.c. embryos, vessel networks along anatomical boundaries in the periorbital (po), lower jaw (Ij) as well as in the neck (ne) region contained FLT4 positive endothelia. Similar stuctures were present in the pericardial space and throughout the subcutaneous (sc) tissue. Notably, in contrast to FLT4 negative vessels, all FLT4 positive vessels were devoid of blood cells in their lumen. These expression patterns suggest that FLT4 becomes confined to the endothelia of lymphatic vessels at this time of development. An additional site where we observed FLT4 expression, was in the sinusoids of the developing bone marrow (bm).

Photographs of a transverse section of the upper thorax of a 16.5 day p.c. embryo hybridized with the FLT4 probe are shown in panels C and D of Figure 4. The section shown in C has been stained with hematoxylin-eosin to visualize the different types of vessels in this area. These include the carotic and brachiochepalic arteries (ca, ba), the vena cava (vc) and the thoracic duct, which is smaller in size and lacks surrounding muscular and connective tissue (arrow). A magnification of the region of thoracic duct is shown in panel D, where the FLT4 autoradiographic grains can be seen. Endothelial cells of the thoracic duct as well as a small vessel (v) in the vicinity hybridize with the FLT4 probe.

### EXAMPLE 4

### Analysis of FLT4 mRNA in cultured endothelial cells

The *in situ* hybridization results described in Example 3, showed that FLT4 is expressed in venous endothelial cells and later in lymphatic vessels and some venous endothelial cells, but not in arterial endothelia. In order to see if such regulation was maintained *in vitro*, we studied cultured endothelial cells using Northern blotting and hybridization analysis.

Endothelial cells from human aorta, femoral vein, umbilical vein, and from foreskin microvessels were isolated, cultured and characterized as previously described by Van Hinsberg, ^{(15,16)}. They were used at confluent density after five to eight passages (split ratio 1:3) for the isolation of polyadenylated RNA.

The endothelial cell lines EA·hy926, BCE and LEII did not express FLT4 (data not shown). However, cultured human microvascular, venous and umbilical vein endothelial cells were positive for the FLT4-specific 5.8 and 4.5 kb mRNAs, whereas the aortic endothelial cells were negative (Fig. 5). In contrast, another endothelial receptor tyrosine kinase gene, Tie, was expressed as a 4.4 kb mRNA in all endothelial cell types studied.

### EXAMPLE 5

### FLT4 mRNA in in adult human tissues

The results obtained in Example 3 indicated that the FLT4 mRNA becomes largely confined to the endothelium of lymphatic vessels during development. Because of the potential significance of this finding in humans, we also studied FLT4 in adult human tissues using a human FLT4 probe. The human FLT4 probe used was an EcoRI-SphI fragment covering base pairs 1-595 of the cDNA ⁽³⁾. The von Willebrand factor probe was an EcoRI-Hindlll fragment covering base pairs 1-2334 ⁽¹⁷⁾.

We used routinely fixed material sent for histopathological diagnosis. Normal lung tissue was obtained from a resection of the left inferior lung lobe affected by epidermoid cancer. Mesenterium and mesenterial lymph nodes were obtained from a patient having a colonic adenocarcinoma. A normal lymph node adjacent to the salivary gland was enucleated because of its abnormal size. The tonsils from two patients and the two appendixes had no diagnostic changes. Two lymphangiomyomas and three cystic lymphangiomas were studied with similar results.

For human tissues, which were routine samples fixed with 10% formalin for histopathological diagnosis, the normal *in situ* protocol gave just backround, whereas microwave treatment instead of proteinaase K enabled specific hybridization ^{(18, 19)},

In the mesenterium, lung and appendix lymphatic endothelia (Iv) gave FLT4 signals, while veins (v), arteries (a) and capillaries (c) were negative (Fig. 6A-D and data not shown). To study whether FLT4 is expressed in the HEVs, the tonsils were studied. Indeed, in the tonsils, FLT4 specific autoradiographic grains were detected in some HEVs (E, F).

### EXAMPLE 6

### Analysis of FLT4 mRNA in normal and metastatic lymph node and in lymphangioma

A portion of a human mesenterial lymph node (see Example 5) was analysed for FLT4 expression. The results are shown in Figure 7.

FLT4 is expressed in the lymphatic sinuses (Is) and afferent and efferent lymphatic vessels (data not shown). The same pattern is seen in a lymph node containing adenocarcinoma metastases (C, D). Some HEVs in both normal and metastatic lymph node were also positive. In panel E, FLT4 expression is shown in a cystic lymphangioma (compare with the hematoxylin-eosin stained section in F). Notably, the specificity of FLT4 to lymphatic endothelia is evident from the comparison with the *in situ* signals for von Willebrandt factor in all blood vessels (F).

### EXAMPLE 7

### Localization of FLT4 in fetal endothelial cells

An FLT4 cDNA fragment encoding the 40 carboxy terminal amino acids of the short form was cloned as a 657 bp *Eco*RI-fragment into the pGEX-1IT bacterial expression vector (Pharmacia) in frame with the glutatione-S-transferase coding region. The resulting GST-FLT4 fusion protein was produced in *E.coli* and purified by affinity chromatography using a glutathione-Sepharose 4B column. The purified protein was lyophilized, disolved in PBS, mixed with Freund's adjuvant and used for immunization of rabbits. Antisera were used after the fourth booster immunization.

Tissues from 17 and 20-week-old human fetuses were obtained from legal abortions induced with prostaglandins. The study was approved by the Ethical Committee of the Helsinki University Central Hospital. The gestational age was estimated from the fetal foot length. The fetal tissues were embedded in Tissue-Tek (Miles), frozen immediately and stored at -70 °C.

Anti-FLT4 antiserum was cross-absorbed to a GST-Sepharose column to remove anti-GST-antibodies and then purified by GST-FLT4 affinity chromatography. Several 6 µm-thick cryostat sections of the tissues were fixed with acetone and treated with 0.3% H₂O₂ in methanol for 30 min to block endogenous peroxidase activity. After washing, the sections were incubated with 5% normal swine serum. Sections were then incubated with antibodies against FLT4, washed and bound antibodies were detected with peroxidase-conjugated swine anti-rabbit IgG followed by staining for peroxidase activity using 0.2% 3,3-diaminobenzidine (Amersham) as a substrate. The sections were counterstained in Meyer's hematoxylin.

Anti-FLT4 immunoperoxidase staining of human fetal mesenterium showed FLT4 protein in the endothelium of several vessels (Fig. 4A), while control stainings with antigen-blocked anti-FLT4 antibodies (B) and preimmune sera (C) were negative. For comparison, Figure 4D shows results of staining using an antiserum against the factor VIII-related antigen, which is specific for vascular endothelial cells.

### EXAMPLE 8

### Production of monoclonal antibodies against FLT4

### Fusion I:

Four months old Balb/c male mice were immunized by intraperitoneal injection of the recombinantly produced FLT4 protein (see Example 7) in concentrated medium (150 µg/mouse), emulsified with Freund's complete adjuvant. Booster injections of 150 µg were given at three to four week intervals and a final booster (10 µg FLT4 in PBS administered intraperitoneally) was given after another three-week interval. Four days after the final booster dose, the mice were sacrified and mouse splenic lymphoid cells were fused with SP 2/0 plasmacytoma cells at a 2:1 ratio, respectively.

The fused cells were harvested in 96-well culture plates (NUNC) in Ex-Cell 320 medium (SERALAB) containing 20% fetal calf serum and HAT supplement (hypoxanthine-aminopterin-thymidine; GIBCO, 043-01060H; diluted 50-fold). Cells were cultured at +37°C, ina 5% CO₂ atmosphere. After 10 days, HAT-supplemented medium was changed to HT-supplemented cell culture medium (GIBCO; 043-01065H, diluted 50-fold). HT medium is identical to HAT medium, but lacks aminopterin.

In three weeks specific antibody production was determined by the antigen-specific immunofluorometric assay, IFMA, described in Example 10 The master clones were cloned by limited dilutions as described by Staszewski et al., Yale Journal of Biology and Medicine, 57:865-868 (1984). Positive clones were expanded onto 24-well tissue culture plates (NUNC), recloned, and re-tested by the same method. Positive clones were tested by fluorescence-activated cell sorting (FACS).

The stable clones secreted immunoglobulins belonging to the IgG1 class, except one, which produced Ig probably belonging to class IgA. The subclass of monoclonal antibody was determined using rat monoclonal antibody to mouse subclass as biotin conjugate (SEROTEC) in IFMA.

Balb/c mice were used to produce monoclonal antibodies in ascites fluid. The hybridomas described above were intraperitoneally injected into mice after pretreatment of the animals with pristane (2,6,10,14-tetramethylpentadecan 98%, ALDRICH-CHEMIE D7924 Steinheim, Cat.No. T 2,280-2). 0.5 ml of pristane (i.v.) was injected about two weeks prior to the hybridoma cells. The amount of cells injected were approximately 7.5 to 9 x 10⁶ per mouse. Ascites was collected 10 to 14 days after injection of the hybridomas.

### Fusion II:

Two months old Balb/c mice (female) were immunized by intraperitoneal injection of the recombinantly produced FLT-4 protein (see Example 7) (20 µg/mouse), emulsified with Freund's complete adjuvant. Booster injections of 20 µg were given at three to four week intervals and a final booster (10 µg FLT-4 in PBS administered i.v.) was given after another three-week interval. Four days after the final booster dose, the mice were sacrified and mouse splenic lymphoid cells were fused with SP 2/0 plasmacytoma cells at a 2:1 ratio, respectively.

The fused cells were harvested in 96-well culture plates (FALCON) in OptiMEM 1 (with Glutamax 1, 51985-026, GIBCO BRL) medium containing 20 % fetal calf serum and HAT supplement (hypoxanthine-aminopterin-thymidine; GIBCO BRL 21060-017; diluted 1:50 fold). Cells were cultured at +37 °C, in a 5% CO2 atmosphere. After 10 days, HAT-supplemented medium was changed to HT-supplemented cell culture medium (GIBCO BRL; 41065-012, diluted 1:50-fold). HT-medium is identical to HAT-medium,but lacks aminopterin.

In three weeks specific antibody production was determined by the antigen-specific ImmunoFluoroMetric Assay (IFMA) described in Example 9. The master clones were cloned by limited dilutions as described by Staszewski et al., Yale Journal of Biology and Medicine, 57:865-868 (1984). Positive clones were expanded onto 24-well tissue culture plates (FALCON), recloned, and re-tested by the same method. Positive clones were tested by fluorescence-activated cell sorting (FACS).

The 2E11 and 6B2 clones secreted immunoglobulins belonging to the IgG₁ class, 2B12 clones produced Ig belonging to subclass IgM . The mouse subclass IgG₁ was determined using rat monoclonal antibody against mouse subclass heavy chain as biotin conjugate (SEROTEC) in IFMA and the mouse subclass IgM was determined with Mouse Monoclonal Antibody lsotyping Kit (Dipstick Format) (19663-012, Life Technologies Inc.).

### EXAMPLE 9

### Specificity of monoclonal antibodies against FLT4

### Fusion I antibodies:

The extracellular domain of FLT4 described in Example 7, was labelled according to Mukkala et al., in *Anal.Biochem*. 176(2):319-325, 1989 , with the following modification: a 250 times molar excess of isothiocyanate DTTA-Eu (N1 chelate, WALLAC, Finland) was added to the FLT4 solution (0.5 mg/ml in PBS) and the pH was adjusted to about 9 by adding 0.5 mol/L sodium carbonate buffer, pH 9.8. The labelling was performed overnight at +4°C. Unbound label was removed using PD-10 (PHARMACIA, Sweden) with TSA buffer (50 mmol/L Tris-HCl, pH 7.8 containing 0.15 mol/l NaCl) as eluent.

After purification, 1 mg/ml bovine serum albumin (BSA) was added to the labelled FLT4 and the label was stored at +4°C. The number of europium ions incorporated per FLT4 molecule was 1.9, as determined by measuring the fluorescence in a ratio to that of known EuCl₃ standards (Hemmilä et al., *Anal.Biochem*., 137:335-343, 1984).

The antibodies produced in Example 8, were screened using a Sandwich type immunofluorometric assay using microtitration strip wells (NUNC, polysorb) coated with rabbid anti-mouse Ig (Z 259, DAKOPATTS). The precoated wells were washed once by Platewash 1296-024 (WALLAC) with DELFIA wash solution. The DELFIA assay buffer was used as a dilution buffer for cell culture supernatants and for serum of the spleenectomized mouse (at dilutions between 1:1000 to 1: 100 000) used as positive control in the preliminary screening assay.

An overnight incubation at +4°C (or alternatively for 2 hours at room temperature) was begun by shaking on a Plateshake shaker (1296-001, WALLAC) for 5 min followed by washing four times with wash solution as described above.

The europium-labelled FLT4 was added at a dilution of 1:500 in 100 µl of the assay buffer. After 5 min on a Plateshake shaker and one hour incubation at RT the strips were washed as described above.

Enhancement solution (DELFIA) was added at 200 µl/well. The plates were then shaken for 5 min an a Plateshake shaker and the intensity of fluorescence was measured by ARCUS-1230 (WALLAC) for 10-15 min. (Lövgren et al., In: Collins W.P. (Ed) Alternative Immunoassays. John Wiley & Sons Ltd, 1985; pp. 203-216).

The resulting.monoclonal antibodies against FLT4 and corresponding FACS results are summarized in Table 2.

**TABLE 2**

| Mab clones | LTR%^{a)} | NEO%b) | DELFIA-counts |
|---|---|---|---|
| 1B1 | 67,3 | 1 | 20625 |
| 1B1D11 | 75 | 1,2 | 19694 |
| 1B1F8 | 76,1 | 1,4 | 18580 |
| 4F6 | 69,9 | 1,2 | 23229 |
| 4F6B8G12 | 75 | 0,3 | 24374 |
| 4F6B8H11 | 75,9 | 0,3 | 28281 |
| 4F6B8E12 | 74,8 | 0,4 | 27097 |
| 4F6B8G10 | 75,3 | 0,4 | 26063 |
| 9D9 | 45,1 | 0,75 | 17316 |
| 9D9D10 | 71,7 | 2,3 | 18230 |
| 9D9F9 | 73 | 1,8 | 11904 |
| 9D9G6 | 74,3 | 2,9 | 16743 |
| 9D9G7 | 70,7 | 1,3 | 17009 |
| 10E4 | 24,2 | 1,4 | 39202 |
| 10E4B10E12 | 32,3 | 0,3 | 42490 |
| 10E4B10G10 | 36,5 | 0,3 | 54815 |
| 10E4B10F12 | 45,6 | 0,4 | 43909 |
| 10E4B10G12 | 45,7 | 0,5 | 35576 |
| 11G2 | 30,2 | 1,6 | 11304 |
| 11G2D12 | 74,4 | 1,5 | 14660 |
| 11G2G9 | 74,2 | 0,9 | 10283 |
| 11G2H7 | 74,4 | 2,1 | 25382 |

| | | | |
|---|---|---|---|
| a) FACS results wiht LTR transfected cells | | | |
| b) FACS results wiht NEO cells (control) | | | |

One clone, designated anti-FLT4 9D9F9 was found to stably secrete monoclonal antibody which was determined to be of immunoglobulin class IgG1 by IFMA. Hybridoma 9d9f9 was deposited with the German Collection of Microorganisms and Cell Cultures, Department of Human and Animal Cell Cultures and Viruses, Mascheroder Weg 1b, 3300 Braunschweig, Germany, March 23, 1995, and given accession No. ACC2210.

### Fusion II antibodies:

The extracellular domain of FLT-4 described in Example 7, was labelled according to Mukkala et al., in Anal.Biochem. 176(2): 319-325, 1989, with the following modification: a 250 times molar excess of isothiocyanate DTTA-Eu (N1 chelate, Wallac, Finland) was added to the FLT-4 solution (0.5 mg/ml in PBS) and the pH was adjusted to about 9 by adding 0.5 mol/L sodium carbonate buffer, pH 9.8. The labelling was performed overnight at +4°C. Unbound label was removed using PD-10 (PHARMACIA) with TSA buffer (50 mmol/L Tris-HCl, pH 7.8 containing 0.15 mol/L NaCl) as eluent.

After purification, 1 mg/ml bovine serum albumin (BSA) was added to the labelled FLT-4 and the label was stored at +4°C. The number of europium ions incorporated per FLT-4 molecule was 1.9, as determined by measuring the fluorescence in a ratio to that of known EuCl3 standards (Hemmil et al., Anal.Biochem., 137: 335-343, 1984).

The antibodies produced in Example 8, were screened using a FLT-4 specific IFMA using microtitration wells (Nunc, Polysorb) coated with rabbit antimouse Ig (Z 259, DAKO). The precoated wells were washed once with wash solution (Wallac) by using DELFIA Plate wash.

The DELFIA assay buffer was used as dilution buffer for cell culture supernatants (dilution 1:2 in preliminary screening) and for serum of the splenectomized mouse (dilutions 1:1 000 to 1:100 000) which was used as positive control. As standard the purified antiFLT-4 9D9F9 (mouse subclass IgG1) was used at concentrations between 1.0 ng/ml and 250 ng/ml. Samples were first shaken at room temperature for five minutes on Plate shake (Wallac) and then incubated approx. 18 hours at +4°C. The frames were first washed four times, then the Eu-labelled FLT-4 (1:2000, in 100 µl assay buffer) was added and finally the frames were incubated for one hour at room temperature. After washing as described the enhancement solution (200 µl/well, Wallac) was added and the frames were shaken for 5 minutes on Plate shake. The intensity of fluorescence was measured by ARCUS-1230 (Wallac).

The resulting monoclonal antibodies against FLT-4 and corresponding results are summarized in Table 3.

A standard curve for quantitation of antiFLT-4 antibodies was made by using affinity purified antiFLT-4 9D9F9. The linear range reached from 1.0 ng/ml to 250 ng/ml.

Cell lysate of NIH 3T3 cells cotranfected with pLTRFLT4 construct expressing full-length FLT4 on the surface was electrophoresed in 6.5% SDS-PAGE, proteins were transfered onto nitrocellulose nitrate membrane (0.45 µm, SCHLEICHER & SCHUELL) and immunoblotted with Mab cell culture supernatants (1:10, 50 mmol/L TRIS - 40 mmol/L glycine buffer containing methanol 4%, SDS 0.04%). The specificity of Mab was detected using incubation with HRP-conjugated rabbit antimouse lg ( P 161, DAKO, diluted 1:1000 in 20 mmol/L TRIS buffer pH 7.5 containing 150 mmol/L saline, 5% milk powder) and ECL (Enhanced chemiluminescence, AMERSHAM).

**TABLE 3.**

| Mab clones | LTR %a) | NEOb) | approx.Mab prod. ng/ml/106 cells | WB |
|---|---|---|---|---|
| 2B12E10 | 39.5 | 6.0 | 440 | + |
| 2E11D11 | 44.6 | 8.8 | 110 | + |
| 2E11F9 | 49.5 | 4.5 | 100 | + |
| 2E11F12 | 46.0 | 4.1 | 180 | + |
| 2E11G8 | 41.2 | 7.8 | 160 | + |
| | | | | |
| 6B2E12 | NF | NF | 1390 | + |
| 6B2F8 | NF | NF | 470 | + |
| 6B2G6 | NF | NF | 630 | + |
| 6B2H5 | NF | NF | 740 | + |
| 6B2H8 | NF | NF | 1800 | + |

| | | | | |
|---|---|---|---|---|
| a) FACS results with LTR transfected cells | | | | |
| b) FACS results with NEO cells (control) NF not functioning in FACS | | | | |
| c) quantitation of Mab production based on affinity purified antiFLT 9D9F9 antibody used as standard | | | | |

As is evident from the foregoing, antibodies according to the present invention are useful in the diagnosis and identification of lymphatic vessels, lymphatic endothelial cells, high endothelial venules, lymphangiomas, metastatic lymph nodes and other disease states of the lymphatic system, the detection and monitoring of metastatic spread, in the stimulation and inhibition of endothelial cells of lymphatic vessels and high endothelial venules, in the introduction of molecules selectively into endothelial cells and in the imaging of lymphatic vessels and their disease states. Other uses of the presently-claimed subject matter are apparent to the skilled artisan.

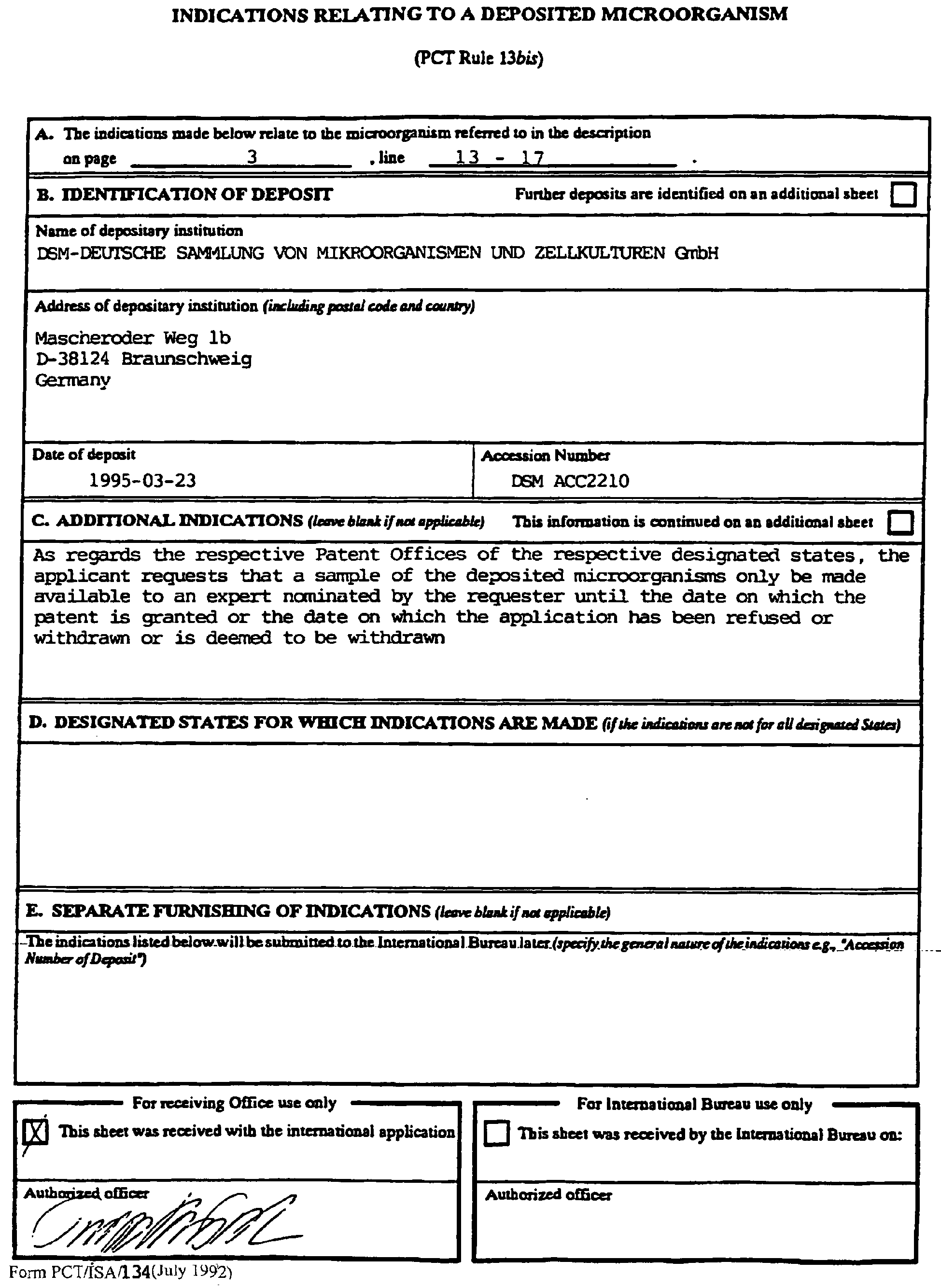
INDICATIONS RELATING TO A DEPOSITED MICROORGANISM

(PCT Rule 13bis)

A. The indications made below relate to the microorganism referred to in the description
on page 3 , line 13 - 17 .

B. IDENTIFICATION OF DEPOSIT | Further deposits are identified on an additional sheet ☐

Name of depositary institution
DSM-DEUTSCHE SAMMLUNG VON MIKROORGANISMEN UND ZELLKULTUREN GmbH

Address of depositary institution (including postal code and country)

Mascheroder Weg 1b
D-38124 Braunschweig
Germany

Date of deposit | Accession Number
1995-03-23 | DSM ACC2210

C. ADDITIONAL INDICATIONS (leave blank if not applicable) | This information is continued on an additional sheet ☐

As regards the respective Patent Offices of the respective designated states, the
applicant requests that a sample of the deposited microorganisms only be made
available to an expert nominated by the requester until the date on which the
patent is granted or the date on which the application has been refused or
withdrawn or is deemed to be withdrawn

D. DESIGNATED STATES FOR WHICH INDICATIONS ARE MADE (if the indications are not for all designated States)

E. SEPARATE FURNISHING OF INDICATIONS (leave blank if not applicable)

The indications listed below will be submitted to the International Bureau later (specify the general nature of the indications e.g., "Accession
Number of Deposit")

For receiving Office use only | For International Bureau use only

☒ This sheet was received with the international application | ☐ This sheet was received by the International Bureau on:

Authorized officer | Authorized officer
 |

Form PCT/ISA/134 (July 1992)

### SEQUENCE LISTING

(1) GENERAL INFORMATION:
   (i) APPLICANT:
      (A) NAME: Alitalo, Kari
      (B) STREET: Nyyrikintie 4 A
      (C) CITY: ESPOO
      (E) COUNTRY: FINLAND
      (F) POSTAL CODE (ZIP): FIN-02100

      (A) NAME: Kaipainen, Arja
      (B) STREET: Messeniuksenkatu 7 A 9
      (C) CITY: HELSINKI
      (E) COUNTRY: FINLAND
      (F) POSTAL CODE (ZIP): FIN-00250

      (A) NAME: Korhonen, Jaana
      (B) STREET: Agricolankatu 7 C 68
      (C) CITY: HELSINKI
      (E) COUNTRY: FINLAND
      (F) POSTAL CODE (ZIP): FIN-00530

      (A) NAME: Mustonen, Tuija
      (B) STREET: Pihlajatie 8 A 2
      (C) CITY: HELSINKI
      (E) COUNTRY: FINLAND
      -(F) POSTAL CODE (ZIP): FIN-00270

      (A) NAME: Pajusola, Katri
      (B) STREET: Kasteholmantie 4 A 8
      (C) CITY: HELSINKI
      (E) COUNTRY: FINLAND
      (F) POSTAL CODE (ZIP): FIN-00900

      (A) NAME: Matikainen, Marja-Terttu
      (B) STREET: Lankkistentanhua 12
      (C) CITY: MXNÄMÄKI
      (E) COUNTRY: FINLAND
      (F) POSTAL CODE (ZIP): FIN-23100

      (A) NAME: Karnani, Paivi
      (B) STREET: Mullintie 10 B 62
      (C) CITY: TURKU
      (E) COUNTRY: FINLAND
      (F) POSTAL CODE (ZIP): FIN-20300
   (ii) TITLE OF INVENTION: FLT4 RECEPTOR TYROSINE KINASE AND ITS USE IN DIAGNOSIS AND THERAPY
   (iii) NUMBER OF SEQUENCES: 1
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.25 (EPO)
   (v) CURRENT APPLICATION DATA: APPLICATION NUMBER: WO TO BE ASSIGNED
   (vi) PRIOR APPLICATION DATA:
      (A) APPLICATION NUMBER: US 08/257754
      (B) FILING DATE: 9-JUN-1994
(2) INFORMATION FOR SEQ ID NO: 1:

CCACGCGCAG CGGCCGGAG ATG CAG CGG GGC GCC GCG CTG TGC CTG CGA CTG
52

1               5                   10

TGG CTC TGC CTG GGA CTC CTG GAC GGC CTG GTG AGT GGC TAC TCC ATG
100

            15                  20                  25

ACC CCC CCG ACC TTG AAC ATC ACG GAG GAG TCA CAC GTC ATC GAC ACC
148

        30                  35                  40

GGT GAC AGC CTG TCC ATC TCC TGC AGG GGA CAG CAC CCC CTC GAG TGG
196

    45                  50                  55

GCT TGG CCA GGA GCT CAG GAG GCG CCA GCC ACC GGA GAC AAG GAC AGC
244

60                  65                  70                  75

GAG GAC ACG GGG GTG GTG CGA GAC TGC GAG GGC ACA GAC GCC AGG CCC
292

                80                  85                  90

TAC TGC AAG GTG TTG CTG CTG CAC GAG GTA CAT GCC AAC GAC ACA GGC
340

            95                  100                 105

AGC TAC GTC TGC TAC TAC AAG TAC ATC AAG GCA CGC ATC GAG GGC ACC
388

        110                 115                 120

ACG GCC GCC AGC TCC TAC GTG TTC GTG AGA GAC TTT GAG CAG CCA TTC
436

    125                 130                 135

ATC AAC AAG CCT GAC ACG CTC TTG GTC AAC AGG AAG GAC GCC ATG TGG
484

140                 145                 150                 155

GTG CCC TGT CTG GTG TCC ATC CCC GGC CTC AAT GTC ACG CTG CGC TCG
532

                160                 165                 170

CAA AGC TCG GTG CTG TGG CCA GAC GGG CAG GAG GTG GTG TGG GAT GAC
580

            175                 180                 185

CGG CGG GGC ATG CTC GTG TCC ACG CCA CTG CTG CAC GAT GCC CTG TAC
628

        190                 195                 200

CTG CAG TGC GAG ACC ACC TGG GGA GAC CAG GAC TTC CTT TCC AAC CCC
676

    205                 210                 215

TTC CTG GTG CAC ATC ACA GGC AAC GAG CTC TAT GAC ATC CAG CTG TTG
724

220                 225                 230                 235

CCC AGG AAG TCG CTG GAG CTG CTG GTA GGG GAG AAG CTG GTC CTG AAC
772

                240                 245                 250

TGC ACC GTG TGG GCT GAG TTT AAC TCA GGT GTC ACC TTT GAC TGG GAC
820

            255                 260                 265

TAC CCA GGG AAG CAG GCA GAG CGG GGT AAG TGG GTG CCC GAG CGA CGC
868

        270                 275                 280

TCC CAG CAG ACC CAC ACA GAA CTC TCC AGC ATC CTG ACC ATC CAC AAC
916

    285                 290                 295

GTC AGC CAG CAC GAC CTG GGC TCG TAT GTG TGC AAG GCC AAC AAC GGC
964

300                 305                 310                 315

ATC CAG CGA TTT CGG GAG AGC ACC GAG GTC ATT GTG CAT GAA AAT CCC
1012

                320                 325                 330

TTC ATC AGC GTC GAG TGG CTC AAA GGA CCC ATC CTG GAG GCC ACG GCA
1060

            335                 340                 345

GGA GAC GAG CTG GTG AAG CTG CCC GTG AAG CTG GCA GCG TAC CCC CCG
1108

        350                 355                 360

CCC GAG TTC CAG TGG TAC AAG GAT GGA AAG GCA CTG TCC GGG CGC CAC
1156

    365                 370                 375

AGT CCA CAT GCC CTG GTG CTC AAG GAG GTG ACA GAG GCC AGC ACA GGC
1204

380                 385                 390                 395

ACC TAC ACC CTC GCC CTG TGG AAC TCC GCT GCT GGC CTG AGG CGC AAC
1252

                400                 405                 410

ATC AGC CTG GAG CTG GTG GTG AAT GTG CCC CCC CAG ATA CAT GAG AAG
1300

            415                 420                 425

GAG GCC TCC TCC CCC AGC ATC TAC TCG CGT CAC AGC CGC CAG GCC CTC
1348

        430                 435                 440

ACC TGC ACG GCC TAC GGG GTG CCC CTG CCT CTC AGC ATC CAG TGG CAC
1396

    445                 450                 455

TGG CGG CCC TGG ACA CCC TGC AAG ATG TTT GCC CAG CGT AGT CTC CGG
1444

460                 465                 470                 475

CGG CGG CAG CAG CAA GAC CTC ATG CCA CAG TGC CGT GAC TGG AGG GCG
1492

                480                 485                 490

GTG ACC ACG CAG GAT GCC GTG AAC CCC ATC GAG AGC CTG GAC ACC TGG
1540

            495                 500                 505

ACC GAG TTT GTG GAG GGA AAG AAT AAG ACT GTG AGC AAG CTG GTG ATC
1588

        510                 515                 520

CAG AAT GCC AAC GTG TCT GCC ATG TAC AAG TGT GTG GTC TCC AAC AAG
1636

    525                 530                 535

GTG GGC CAG GAT GAG CGG CTC ATC TAC TTC TAT GTG ACC ACC ATC CCC
1684

540                 545                 550                 555

GAC GGC TTC ACC ATC GAA TCC AAG CCA TCC GAG GAG CTA CTA GAG GGC
1732

                560                 565                 570

CAG CCG GTG CTC CTG AGC TGC CAA GCC GAC AGC TAC AAG TAC GAG CAT
1780

            575                 580                 585

CTG CGC TGG TAC CGC CTC AAC CTG TCC ACG CTG CAC GAT GCG CAC GGG
1828

        590                 595                 600

AAC CCG CTT CTG CTC GAC TGC AAG AAC GTG CAT CTG TTC GCC ACC CCT
1876

    605                 610                 615

CTG GCC GCC AGC CTG GAG GAG GTG GCA CCT GGG GCG CGC CAC GCC ACG
1924

620                 625                 630                 635

CTC AGC CTG AGT ATC CCC CGC GTC GCG CCC GAG CAC GAG GGC CAC TAT
1972

                640                 645                 650

GTG TGC GAA GTG CAA GAC CGG CGC AGC CAT GAC AAG CAC TGC CAC AAG
2020

            655                 660                 665

AAG TAC CTG TCG GTG CAG GCC CTG GAA GCC CCT CGG CTC ACG CAG AAC
2068

        670                 675                 680

TTG ACC GAC CTC CTG GTG AAC GTG AGC GAC TCG CTG GAG ATG CAG TGC
2116

    685                 690                 695

TTG GTG GCC GGA GCG CAC GCG CCC AGC ATC GTG TGG TAC AAA GAC GAG
2164

700                 705                 710                 715

AGG CTG CTG GAG GAA AAG TCT GGA GTC GAC TTG GCG GAC TCC AAC CAG
2212

                720                 725                 730

AAG CTG AGC ATC CAG CGC GTG CGC GAG GAG GAT GCG GGA CGC TAT CTG
2260

            735                 740                 745

TGC AGC GTG TGC AAC GCC AAG GGC TGC GTC AAC TCC TCC GCC AGC GTG
2308

        750                 755                 760

GCC GTG GAA GGC TCC GAG GAT AAG GGC AGC ATG GAG ATC GTG ATC CTT
2356

    765                 770                 775

GTC GGT ACC GGC GTC ATC GCT GTC TTC TTC TGG GTC CTC CTC CTC CTC
2404

780                 785                 790                 795

ATC TTC TGT AAC ATG AGG AGG CCG GCC CAC GCA GAC ATC AAG ACG GGC
2452

                800                 805                 810

TAC CTG TCC ATC ATC ATG GAC CCC GGG GAG GTG CCT CTG GAG GAG CAA
2500

            815                 820                 825

TGC GAA TAC CTG TCC TAC GAT GCC AGC CAG TGG GAA TTC CCC CGA GAG
2548

        830                 835                 840

CGG CTG CAC CTG GGG AGA GTG CTC GGC TAC GGC GCC TTC GGG AAG GTG
2596

    845                 850                 855

GTG GAA GCC TCC GCT TTC GGC ATC CAC AAG GGC AGC AGC TGT GAC ACC
2644

860                 865                 870                 875

GTG GCC GTG AAA ATG CTG AAA GAG GGC GCC ACG GCC AGC GAG CAC CGC
2692

                880                 885                 890

GCG CTG ATG TCG GAG CTC AAG ATC CTC ATT CAC ATC GGC AAC CAC CTC
2740

            895                 900                 905

AAC GTG GTC AAC CTC CTC GGG GCG TGC ACC AAG CCG CAG GGC CCC CTC
2788

        910                 915                 920

ATG GTG ATC GTG GAG TTC TGC AAG TAC GGC AAC CTC TCC AAC TTC CTG
2836

    925                 930                 935

CGC GCC AAG CGG GAC GCC TTC AGC CCC TGC GCG GAG AAG TCT CCC GAG
2884

940                 945                 950                 955

CAG CGC GGA CGC TTC CGC GCC ATG GTG GAG CTC GCC AGG CTG GAT CGG
2932

                960                 965                 970

AGG CGG CCG GGG AGC AGC GAC AGG GTC CTC TTC GCG CGG TTC TCG AAG
2980

            975                 980                 985

ACC GAG GGC GGA GCG AGG CGG GCT TCT CCA GAC CAA GAA GCT GAG GAC
3028

        990                 995                 1000

CTG TGG CTG AGC CCG CTG ACC ATG GAA GAT CTT GTC TGC TAC AGC TTC
3076

    1005                1010                1015

CAG GTG GCC AGA GGG ATG GAG TTC CTG GCT TCC CGA AAG TGC ATC CAC
3124

1020                1025                1030                1035

AGA GAC CTG GCT GCT CGG AAC ATT CTG CTG TCG GAA AGC GAC GTG GTG
3172

                1040                1045                1050

AAG ATC TGT GAC TTT GGC CTT GCC CGG GAC ATC TAC AAA GAC CCT GAC
3220

            1055                1060                1065

TAC GTC CGC AAG GGC AGT GCC CGG CTG CCC CTG AAG TGG ATG GCC CCT
3268

        1070                1075                1080

GAA AGC ATC TTC GAC AAG GTG TAC ACC ACG CAG AGT GAC GTG TGG TCC
3316

    1085                1090                1095

TTT GGG GTG CTT CTC TGG GAG ATC TTC TCT CTG GGG GCC TCC CCG TAC
3364

1100                1105                1110                1115

CCT GGG GTG CAG ATC AAT GAG GAG TTC TGC CAG CGG CTG AGA GAC GGC
3412

                1120                1125                1130

ACA AGG ATG AGG GCC CCG GAG CTG GCC ACT CCC GCC ATA CGC CGC ATC
3460

            1135                1140                1145

ATG CTG AAC TGC TGG TCC GGA GAC CCC AAG GCG AGA CCT GCA TTC TCG
3508

        1150                1155                1160

GAG CTG GTG GAG ATC CTG GGG GAC CTG CTC CAG GGC AGG GGC CTG CAA
3556

    1165                1170                1175

GAG GAA GAG GAG GTC TGC ATG GCC CCG CGC AGC TCT CAG AGC TCA GAA
3604

1180                1185                1190                1195

GAG GGC AGC TTC TCG CAG GTG TCC ACC ATG GCC CTA CAC ATC GCC CAG
3652

                1200                1205                1210

GCT GAC GCT GAG GAC AGC CCG CCA AGC CTG CAG CGC CAC AGC CTG GCC
3700

            1215                1220                1225

GCC AGG TAT TAC AAC TGG GTG TCC TTT CCC GGG TGC CTG GCC AGA GGG
3748

        1230                1235                1240

GCT GAG ACC CGT GGT TCC TCC AGG ATG AAG ACA TTT GAG GAA TTC CCC
3796

    1245                1250                1255

ATG ACC CCA ACG ACC TAC AAA GGC TCT GTG GAC AAC CAG ACA GAC AGT
3844

1260                1265                1270                1275

GGG ATG GTG CTG GCC TCG GAG GAG TTT GAG CAG ATA GAG AGC AGG CAT
3892

                1280                1285                1290

AGA CAA GAA AGC GGC TTC AGG TAGCTGAAGC AGAGAGAGAG AAGGCAGCAT
3943

            1295

ACGTCAGCAT TTTCTTCTCT GCACTTATAA GAAAGATCAA AGACTTTAAG ACTTTCGCTA
4003

TTTCTTCTAC TGCTATCTAC TACAAACTTC AAAGAGGAAC CAGGAGGACA AGAGGAGCAT
4063

GAAAGTGGAC AAGGAGTGTG ACCACTGAAG CACCACAGGG AAGGGGTTAG GCCTCCGGAT
4123

GACTGCGGGC AGGCCTGGAT AATATCCAGC CTCCCACAAG AAGCTGGTGG AGCAGAGTGT
4183

TCCCTGACTC CT
4195

### REFERENCES

1. Sabin, F.R. 1909. The lymphatic system in human embryos, with consideration of the morphology of the system as a whole. *Am. J*. *Anat.* 9:43.
2. van der Putte, S.C.J. 1975. The development of the lymphatic system in man. *Adv. Anat. Embryol. Cell Biol.* 51:3.
3. Pajusola, K., O. Aprelikova, J. Korhonen, A. Kaipainen, L. Pertovaara, R. Alitalo, and K. Alitalo. 1992. FLT4 receptor tyrosine kinase contains seven immunoglobulin-like loops and is expressed in multiple human tissues and cell lines. *Cancer Res.* 52:5738.
4. Kaipainen, A., J. Korhonen, K. Pajusola, O. Aprelikova, M.G. Persico, B.I. Terman, and K. Alitalo. 1993. The Related FLT4, FLT1 and KDR receptor tyrosine kinases show distinct expression patterns in human fetal endothelial cells. *J. Exp. Med.* 178:2077.
5. Galland, F., A. Karamysheva, M.-J. Pebusque, J.-P. Borg, R. Rottapel, P. Dubreuil, O. Rosnet, and D. Birnbaum. 1993. The *FLT4* gene encodes a transmembrane tyrosine kinase related to the vascular endothelial growth factor receptor. *Oncogene.* 8:1233.
6. Millauer, B., S. Wizigmann-Voos, H. Schnürch. R. Martinez, N.-P.H. Moller, W. Risau, and A. Ullrich. 1993. High affinity VEGF binding and developmental expression suggest Flk-1 as a major regulator of vasculogenesis and angiogenesis. *Cell.* 72:835.
7. Yamaguchi, T.P., D. Dumont, R.A. Conlon, M.L. Breitman, and J. Rossant. 1993. *flk-1,* an *fit*-related tyrosine kinase is an early marker for edothelial cell precursors. *Development*. 118:489.
8. Peters, K.G., C. De Vries, and L.T. Williams. 1993. Vascular endothelial growth factor receptor expression during embryogenesis and tissue repair suggests a role in endothelial differentiation and blood vessel growth. *Proc. Natl. Acad. Sci. USA* 90:8915.
9. Finnerty, H., K. Kelleher, G. Morris E., K. Bean, D. Merberg, R. Kritz, J. Morris C., H. Sookdeo, K.J. Turner, and C.R. Wood 1993. Molecular cloning of murine FLT and FLT4. *Oncogene.* 8:2293.
10. Korhonen, J., A. Polvi, J. Partanen, and K. Alitalo. 1993. The mouse *tie* receptor tyrosine kinase gene: expression during embryonic angiogenesis. *Oncogene.* 8:395.
11. Chomczynski, P., and N. Sacchi. 1987. Single-step method of RNA isolation by acid guanidium thiocynate-phenol-chloroform extraction. *Anal. Biochem.* 162:156.
12. Tokunaga, K., H. Taniguchi, K. Yoda, M. Shimizu, and S. Sakiyama. 1986. Nucleotide sequence of a full-length cDNA for mouse cytoskeletal beta-acting m RNA. *Nucleic. Acid. Res.* 14:2829.
13. Wilkinson, D.G., J.A. Bailes, J.E. Champion, and A.P. McMahon. 1987. A molecular analysis of mouse development from 8 to 10 days post coitum detects changes only in embryonic globin expression. *Development.* 99:493.
14. Wilkinson, D.G., J.A. Bailes, and A.P. McMahon. 1987. Expression of the proto-oncogene int-1 is restricted to specific neural cells in the developing mouse embryo. *Cell.* 50:79.
15. Van Hinsberg, V.W.M., D. Binnema, M.A. Scheffer, E.D. Sprengers, T. Kooistra, and D.C. Rijken. 1987. Production of plasminogen activators and inhibitors by serially propagated endothelial cells from adult human blood vessels. *Arteriosclerosis*. 7:389.
16. Van Hinsberg, V.W.M., M.A. Scheffer, and T. Kooistra. 1987. Effect of thrombin on the production of plasminogen activators and PA inhibitor-1 by human foreskin microvascular endothelial cells. *Thromb. Haemostas*. 57:148.
17. Bonthron, D.T., E.C. Orr, L.M. Mitsock, D. Ginsberg, R.I. Handin, and S.H. Orkin. 1986. Nucleotide sequence of pre-pro-von Willebrand factor cDNA. *Nucleic Acids Res.* 141:7125.
18. Shi, E., M. Kan, J. Xu, and W.L. McKeehan. 1991. 16-kilodalton heparin binding (fibroblast) growth factor type one appers in a stable 40-kilodalton complex after receptor-dependent internalization. *J. Biol. Chem.* 266:5774.
19. Catoretti, G., M.H.G. Becker, G. Key, M. Duchrow, C. Schlüter. J. Galle, and J. Gerdest. 1992. Monoclonal antibodies against recombinant parts of the Ki-67 antigen (MIB 1 and MIB 3) detect proliferating cells in microwave-processed formalin-fixed paraffin section. *J. of Pathol.* 168:357.

## Claims

1. Use of a monoclonal antibody directed against the extracellular domain of the FLT4-receptor tyrosine kinase for the preparation of a diagnostic composition for imaging lymphatic vessels, lymph nodes or high endothelial venules (HEVs).

2. Use of a monoclonal antibody directed against the extracellular domain of the FLT4-receptor tyrosine kinase for the preparation of a diagnostic composition for detecting lymphatic tissue, lymphatic vessels or high endothelial venules (HEVs).

3. The use of claim 2, wherein the lymphatic tissue to be detected is lymph node tissue.

4. Use of a monoclonal antibody directed against the extracellular domain of the FLT4-receptor tyrosine kinase for the preparation of pharmaceutical composition for inhibiting the FLT-4 mediated lymphatic vascularization which is associated with a disease selected from the group consisting: metastatic cancers, lymphomas, lymphangiomas, inflammation (chronic or acute), infections and immunological diseases.

5. The use of any one of claims 1 to 4, wherein said monoclonal antibody is an anti-FLT4 monoclonal antibody produced by a hybridoma cell line deposited as DSM ACC 2210.

6. A method for in vitro imaging lymphatic vessels in a tissue sample, comprising the steps of:
(a) applying a detectably labeled anti-FLT4 antibody to said tissue sample suspected of containing lymphatic vessels; and
(b) detecting said detectably-labeled anti-FLT4 antibody bound to said tissue sample.

7. A method for diagnosing diseases **characterized by** changes in lymphatic vessels and HEVs, comprising the steps of:
(a) exposing a tissue sample obtained from a patient suspected of having a disease **characterized by** changes in lymphatic cells and HEVs to a labeled anti-FLT-4 antibody;
(b) washing said tissue sample; and
(c) detecting the presence of said detectably-labeled anti-FLT-4 antibody in said tissue sample.

## Patentansprüche

1. Verwendung eines monoclonalen Antikörpers, der gegen die extrazelluläre Domäne der FLT4-Rezeptor-Tyrosinkinase gerichtet ist, für die Herstellung eines diagnostischen Mittels zur Darstellung von Lymphgefäßen, Lymphknoten oder Venolen mit hohem Endothel (HEVs).

2. Verwendung eines monoclonalen Antikörpers, der gegen die extrazelluläre Domäne der FLT4-Rezeptor-Tyrosinkinase gerichtet ist, für die Herstellung eines diagnostischen Mittels zum Nachweis von lymphatischem Gewebe, lymphatischen Gefäßen oder Venolen mit hohem Endothel (HEVs).

3. Verwendung nach Anspruch 2, wobei das nachzuweisende lymphatische Gewebe Lymphknotengewebe ist.

4. Verwendung eines monoclonalen Antikörpers, der gegen die extrazelluläre Domäne der FLT4-Rezeptor-Tyrosinkinase gerichtet ist, für die Herstellung eines Arzneimittels zur Verhinderung der FLT4-vermittelten lymphatischen Gefäßneubildung, die assoziiert ist mit einer Krankheit ausgewählt aus der Gruppe bestehend aus: Metastasen-bildendem Krebs, Lymphomen, Lymphangiomen, Entzündung (chronisch oder akut), Infektionen und immunologischen Krankheiten.

5. Verwendung nach einem der Ansprüche 1 bis 4, wobei der monoclonale Antikörper ein monoclonaler Anti-FLT4-Antikörper ist, produziert von einer Hybridomzelllinie, die als DSM ACC 2210 hinterlegt ist.

6. Verfahren zum in vitro-Darstellen von lymphatischen Gefäßen in einer Gewebeprobe, umfassend die Schritte:
(a) Applizieren eines nachweisbar markierten Anti-FLT4-Antikörpers auf die Gewebeprobe, die in Verdacht steht, lymphatische Gefäße zu enthalten; und
(b) Nachweisen des nachweisbar markierten Anti-FLT4-Antikörpers, der an die Gewebeprobe gebunden ist.

7. Verfahren zum Diagnostizieren von Krankheiten, die durch Veränderungen in lymphatischen Gefäßen und HEVs charakterisiert sind, umfassend die Schritte:
(a) Inkontaktbringen einer Gewebeprobe, die von einem Patienten erhalten wurde, der in Verdacht steht, eine Krankheit zu haben, die durch Veränderungen in lymphatischen Zellen und HEVs charakterisiert ist, mit einem markierten Anti-FLT4-Antikörper;
(b) Waschen der Gewebeprobe; und
(c) Nachweisen der Gegenwart des nachweisbar markierten Anti-FLT4-Antikörpers in der Gewebeprobe.

## Revendications

1. Utilisation d'un anticorps monoclonal dirigé contre le domaine extra-cellulaire de la tyrosine kinase du récepteur FLT4 pour la préparation d'une composition diagnostic pour mettre en image les vaisseaux lymphatiques, les noeuds lymphatiques ou les hautes veinules endothéliales (HEVs).

2. Utilisation d'un anticorps monoclonal dirigé contre le domaine extra-cellulaire de la tyrosine kinase du récepteur FLT4 pour la préparation d'une composition diagnostic pour détecter le tissu lymphatique, les vaisseaux lymphatiques ou les hautes veinules endothéliales (HEVs).

3. Utilisation selon la revendication 2, dans laquelle le tissu lymphatique à détecter est le tissu du noeud lymphatique.

4. Utilisation d'un anticorps monoclonal dirigé contre le domaine extra-cellulaire de la tyrosine kinase du récepteur FLT4 pour la préparation d'une composition pharmaceutique pour inhiber la vascularisation lymphatique médiée par FLT4 qui est associée à une maladie choisie dans le groupe constitué des : cancers métastatiques, lymphomes, lymphangiomes, inflammation (chronique ou aiguë), infections et maladies immunologiques.

5. Utilisation selon l'une quelconque des revendications 1 à 4, dans laquelle ledit anticorps monoclonal est un anticorps monoclonal anti-FLT4 produit par une lignée de cellules d'hybridome déposée en tant que DSM ACC 2210.

6. Méthode pour mettre en image in vitro les vaisseaux lymphatiques dans un échantillon de tissu, comprenant les étapes consistant à :
(a) appliquer un anticorps anti-FLT4 marqué de façon détectable au dit échantillon de tissu suspecté de contenir des vaisseaux lymphatiques ; et
(b) détecter ledit anticorps anti-FLT4 marqué de façon détectable lié au dit échantillon de tissu.

7. Méthode pour diagnostiquer les maladies **caractérisées par** des changements dans les vaisseaux lymphatiques et les HEVs, comprenant les étapes consistant à :
(a) exposer un échantillon de tissu obtenu à partir d'un patient suspecté de souffrir d'une maladie **caractérisée par** des changements dans les vaisseaux lymphatiques et les HEVs à un anticorps anti-FLT4 marqué ;
(b) laver ledit échantillon de tissu ; et
(c) détecter la présence dudit anticorps anti-FLT4 marqué de façon détectable dans ledit échantillon de tissu.
